# EUROPEAN PATENT APPLICATION

(11) **EP 4 663 144 A1**
(43) Date of publication of application: **17.12.2025**
(21) Application number: 25176206.8
(22) Date of filing: 13.05.2025
(51) Int. Cl.: A61B 18/14, A61N 1/32, A61B 18/00, A61N 1/06, A61N 1/05, A61B 90/00

(54) **INVASIVE HIGH-FREQUENCY TREATMENT DEVICE THAT CAN DETERMINE THE INITIAL LOCATION OF THE PROCEDURE AT A CONSTANT TIME**

(30) Priority: 10.06.2024 KR 20240074756
(71) Applicant: VIOL Co. Ltd., Bundang-gu Seongnam-si, Gyeonggi-do, 13510 (KR)
(72) Inventor: LEE, Sangjin, 13510 Seongnam-si, Gyeonggi-do (KR); KIM, Jongkil, 13510 Seongnam-si, Gyeonggi-do (KR)
(74) Representative: Zimmermann, Tankred Klaus

(57) **Abstract**

Disclosed is an invasive high-frequency treatment device. The invasive high-frequency treatment device includes multiple micro needles including a first micro needle and a second micro needle, an elevation unit configured to raise the micro needle, and a control circuit configured to control the elevation unit. The control circuit determines a procedure zero point of the elevation unit corresponding to the multiple micro needles in an initialization step of the invasive high-frequency treatment device, and controls the elevation unit to rise at a predetermined height from the procedure zero point in a procedure step of the invasive high-frequency treatment device. Accordingly, it is possible to make consistent the location of the micro needles prior to a procedure regardless of conditions for a design, an assembly, and use.

## Description

### [Technical Field]

The present disclosure relates to a radio frequency treatment device and, particularly, to an invasive high-frequency treatment device capable of performing various treatments (e.g., pain improvement, injury recovery, and wrinkle and scar treatments) by using the principle that an electrical stimulus (e.g., a radio frequency stimulus) is applied by penetrating a fine needle (or a micro needle) into skin to be treated.

### [Background Art]

One of skin procedure methods that are recently in the spotlight is a micro needle procedure. The micro needle procedure is a method of producing fine scars for accelerating the regeneration of the skin by forming a treatment pillar by inserting a very fine needle (or a micro needle) into the skin and simultaneously applying a radio frequency and maximizing the natural healing and regeneration of the skin by inducing the growth factor of a cell as the fine scars heal up.

When a radio frequency is applied to human tissues through the micro needle that plays a role as an electrode for providing a radio frequency stimulus, frictional heat is generated within a tissue surrounding the micro needles due to the radio frequency. Accordingly, skin improvement effects, such as wrinkle reduction and pore tightening, may be achieved because collagen components contract and the formation of new collagen in the dermal layer is promoted.

The micro needle procedure can regenerate collagen and elastic fibers by generating heat (about 40 to 60°C) in a target portion while not causing burns on epidermis. For this reason, the micro needle procedure has been known to be very effective in treatment for acne, a pimple scar, fine wrinkles, deep wrinkles, and a pore reduction and also known to enable obesity treatment by accelerating the combustion of a layer of fat through smooth blood circulation and activating the activities of the lymphatic system.

In general, an invasive high-frequency treatment device that enables the micro needle procedure includes a handpiece, a procedure tip, and a control unit. Micro needles that play a role as an electrode that transfers radio frequency heat energy are disposed at the procedure tip. When the procedure tip is placed at a procedure portion and operated, the micro needles are advanced (e.g., 3 mm or 5 mm) and transfer radio frequency heat energy to the skin layer having a corresponding depth.

In general, the micro needles of the invasive high-frequency treatment device are not exposed to the outside because the micro needles are accommodated within the procedure tip prior to a procedure. However, if the micro needles are exposed to the outside due to a problem, such as a design, an assembly, or use, a person to be treated may feel displeasant because the micro needles first touch the skin of a procedure target portion prior to the procedure.

Furthermore, if the micro needles are accommodated within the procedure tip deeper than a location that is previously predicted, a procedure effect may be halved or a side effect may occur in severe cases because a skin layer having a desired depth is not accurately targeted although the micro needles are advanced.

### [Prior Art Document]

### [Patent Document]

(Patent Document 1) Korean Patent Application Publication No. 10-2015-0060312 (June 3, 2015)
(Patent Document 2) Korean Patent Application Publication No. 10-2021-0055663 (May 17, 2021)

### [Summary of Invention]

### [Technical Problem]

Embodiments of the present disclosure provide an invasive high-frequency treatment device capable of making consistent the location of a micro needle prior to a procedure regardless of conditions for a design, an assembly, and use.

### [Solution to Problem]

According to an aspect of the present disclosure, an invasive high-frequency treatment device includes multiple micro needles including a first micro needle and a second micro needle, an elevation unit configured to raise the micro needle, and a control circuit configured to control the elevation unit. The control circuit determines a procedure zero point of the elevation unit corresponding to the multiple micro needles in an initialization step of the invasive high-frequency treatment device, and controls the elevation unit to rise at a predetermined height from the procedure zero point in a procedure step of the invasive high-frequency treatment device.

In the initialization step, the control circuit may raise the multiple micro needles by controlling the elevation unit, may detect a change in a voltage of at least one of the multiple micro needles, and may determine, as the procedure zero point, a location of the elevation unit corresponding to a location of the micro needle when the change in the voltage is greater than a predetermined reference value based on the results of the detection.

According to an embodiment, the invasive high-frequency treatment device further includes a conductive plate disposed on the multiple micro needles.

According to an embodiment, the invasive high-frequency treatment device further includes a needle cap disposed over the multiple micro needles. The conductive plate may be constructed in a form of an adhesive tape that is attached and fixed to a procedure surface of the needle cap.

According to an embodiment, the invasive high-frequency treatment device further includes a needle cap disposed on the multiple micro needles. The conductive plate may be installed within a tip cover detachably coupled to the needle cap.

The control circuit may include a control unit configured to control the elevation unit, a radio frequency generation unit configured to generate radio frequency power from input power and to apply the generated radio frequency power to the micro needles, and a voltage detection unit configured to apply a voltage that determines an initial procedure location of to the micro needle to the micro needle and to detect a change in the voltage.

The voltage detection unit may output a detection signal indicating that the change in the voltage is greater than the predetermined reference value. The control unit may output, to the elevation unit, an elevation control signal indicative of a stop of the rise of the elevation unit in response to the detection signal. The elevation unit may stop the rise of the elevation unit in response to the elevation control signal and outputs elevation location information indicative of a current location of the elevation unit. The control unit may determine the current location of the elevation unit the rise of which is stopped as the procedure zero point based on the elevation location information.

The control circuit may read a location correction value stored therein and correct the procedure zero point based on the read location correction value.

The control circuit may further include a relay configured to alternatively connect one of the radio frequency generation unit and the voltage detection unit to the micro needle.

The relay may switch to a first location at which the micro needles and the voltage detection unit are electrically connected in the initialization step, and may switch to a second location at which the micro needles and the radio frequency generation unit are electrically connected in the procedure step.

The control circuit may control the invasive high-frequency treatment device to perform the initialization step when the multiple micro needles and the control circuit are newly electrically connected.

### [Advantageous Effects of Invention]

The invasive high-frequency treatment device according to an embodiment of the present disclosure has an effect in that it can make consistent the location of the micro needle prior to a procedure regardless of a change in the initial location of the micro needle according to the type of procedure tip, the state of an assembly with the handpiece, or an assembly state within the handpiece. Accordingly, procedure accuracy and precision can be improved, and discomfort or pain that may occur during the procedure due to a change in the location of the micro needle prior to the procedure can be prevented.

### [Brief Description of Drawings]

FIG. 1 is a front view of an invasive high-frequency treatment device according to an embodiment of the present disclosure.
FIG. 2 is a partial separation diagram of the invasive high-frequency treatment device illustrated in FIG. 1.
FIG. 3 is a schematic diagram schematically illustrating internal components of the invasive high-frequency treatment device according to an embodiment of the present disclosure.
FIG. 4 is an enlarged perspective view illustrating a procedure tip illustrated in FIGS. 1 to 3.
FIG. 5 is an enlarged cutaway perspective view of a portion "A" of the procedure tip illustrated in FIG. 3.
FIG. 6 is a block diagram of components that are necessary to perform an operation of readjusting (or resetting) an initial procedure location and connection relations between corresponding components according to an embodiment of the present disclosure.
FIG. 7 is a flowchart for describing a process of readjusting (or resetting) an initial procedure location of a micro needle, which is performed in the invasive high-frequency treatment device according to an embodiment of the present disclosure.

### [Description of Embodiments]

Hereinafter, embodiments of the present disclosure are described in detail with reference to the accompanying drawings.

Embodiments are provided to more fully explain the present disclosure to a person having ordinary knowledge in the art to which the present disclosure pertains. The following embodiments may be modified in various other forms, and the scope of the present disclosure is not limited to the following embodiments. Rather, these embodiments are provided to make the present disclosure more thorough and complete and to fully convey the spirit of the present disclosure.

Terms used in this specification are used to describe a specific embodiment, and are not intended to limit the present disclosure. Furthermore, in this specification, an expression of the singular number may include an expression of the plural number unless clearly defined otherwise in the context.

In the present application, it is to be understood that a term, such as "include" or "have", is intended to designate that a characteristic, a number, a step, an operation, a component, a part or a combination of them described in the specification is present, and does not exclude the presence or addition possibility of one or more other characteristics, numbers, steps, operations, components, parts, or combinations of them in advance.

Furthermore, when it is said that one component is placed "ahead", "behind", "over", or "under" the other component, the one component may be disposed "ahead", "behind", "over", or "under" the other component by coming into contact with the other component, but it should also be understood that a third component is disposed between the one component and the other component unless specially described. Furthermore, when it is described that one component is "connected" to the other component, it should be understood that the one component is directly connected to the other component, but is indirectly connected to the other component.

The drawings are merely for enabling the spirit of the present disclosure to be understood, and it should not be interpreted that the scope of the present disclosure is limited by the drawings. Furthermore, in the drawings, a relative thickness or length or a relative size may be enlarged for convenience and the clarity of description.

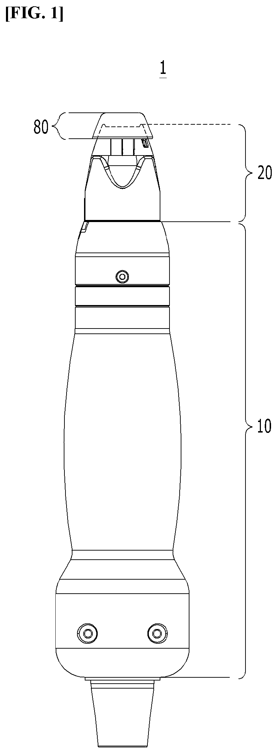

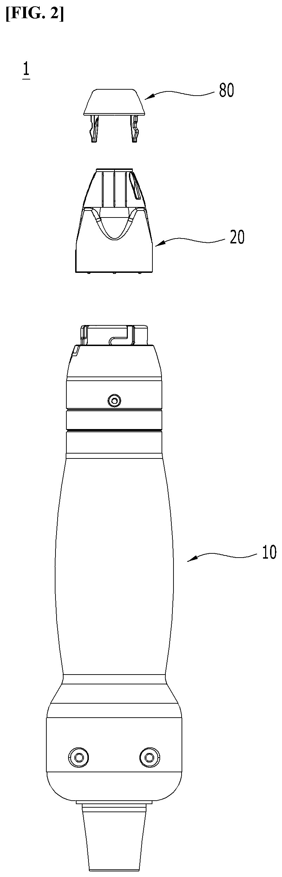

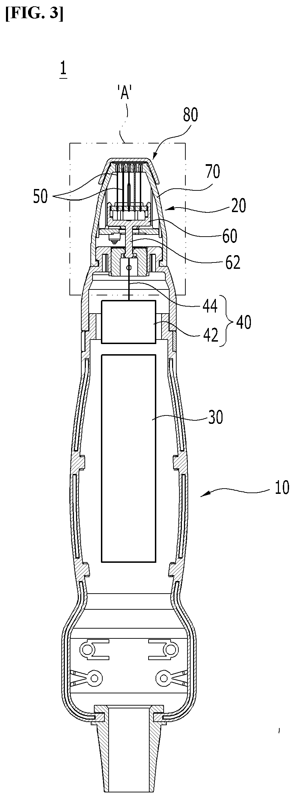

FIG. 1 is a front view of an invasive high-frequency treatment device according to an embodiment of the present disclosure. FIG. 2 is a partial separation diagram of the invasive high-frequency treatment device illustrated in FIG. 1. Furthermore, FIG. 3 is a schematic diagram schematically illustrating internal components of the invasive high-frequency treatment device according to an embodiment of the present disclosure. An overall construction of the radio frequency treatment device according to an embodiment of the present disclosure is schematically described with reference to the drawings.

Referring to FIGS. 1 to 3, the invasive high-frequency treatment device 1 may basically include a handpiece 10 and a procedure tip 20. The procedure tip 20 is coupled to the handpiece 10 in a way to be separable, and may be provided in several types in which the size of the procedure tip and the arrangement state of micro needles 50 are different. Accordingly, an operator (or user) may perform radio frequency treatment by connecting a proper procedure tip 20 to the handpiece 10 in accordance with a procedure portion or the state of the procedure portion.

A control element including a control circuit 30 may be embedded in the handpiece 10. As described above, the control element including the control circuit 30 may be provided in a control main body that is provided separately outside in addition to the type in which the control element is embedded in the handpiece 10. In this case (if the control element is provided in an external control main body), the external control main body and the handpiece 10 may be connected in a wired or wireless way and exchange signals or information.

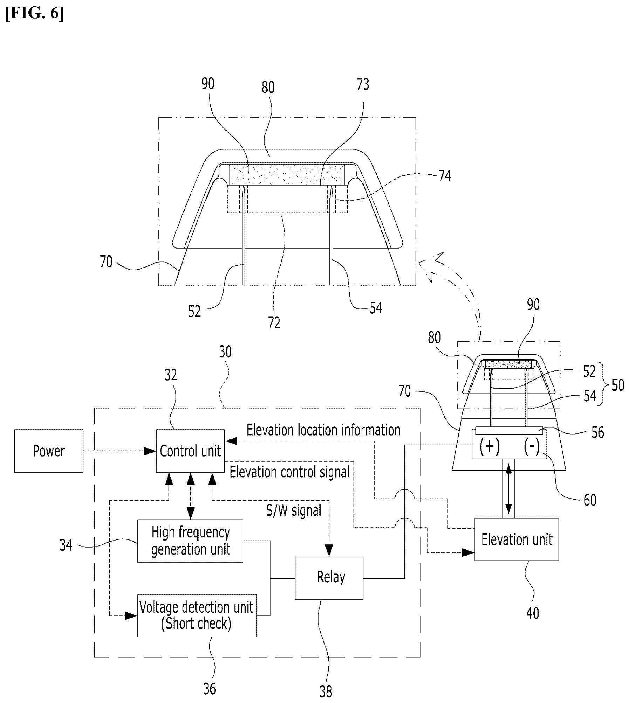

The control circuit 30 includes a control unit 32, a radio frequency generation unit 34, and a voltage detection unit 36 (refer to FIG. 6). As the control unit 32 controls an elevation unit 40, the micro needles 50 included in the procedure tip 20 may rise and fall. The radio frequency generation unit 34 generates a radio frequency from input power and applies the radio frequency to the micro needles 50. Furthermore, the voltage detection unit 36 provides information that is necessary to determine the locations of the micro needles 50 in an initial procedure through a series of such pieces of processing.

The control circuit 30 is described more specifically with reference to FIG. 6.

The procedure tip 20 includes the multiple micro needles 50 and a needle cap 70 that accommodate the multiple micro needles 50. The multiple micro needles 50 penetrates the skin and functions as an electrode that transfers a radio frequency (i.e., radio frequency power) generated by the radio frequency generation unit 34 to a skin layer. The micro needles 50 are fixed to a needle elevation stand 60, and the rise and fall of the micro needles 50 may be synchronized. Furthermore, the needle cap 70 forms an appearance of the procedure tip 20. An accommodation space within the needle cap 70 may accommodate the micro needles 50 and the needle elevation stand 60.

The needle elevation stand 60 may be connected to the elevation unit 40. The elevation unit 40 may include an actuator 42 and an elevation shaft 44. The needle elevation stand 60 may rise and fall within a set range by the elevation unit 40. As a result, the micro needle 50 within the needle cap 70 may protrude from a procedure surface 73 of the needle cap 70, may penetrate the skin, may transfer the radio frequency, and may then return to its initial procedure location within the needle cap 70.

The actuator 42 of the elevation unit 40 may be installed within the handpiece 10. The actuator 42 may be provided in the control main body that is provided separately outside. The actuator 42 may be a motor or a pump, for example. If the actuator 42 is a pump, the elevation shaft 44 may be constructed in the form of a pneumatic cylinder. One end of the elevation shaft 44 may be connected to a shaft unit 62 constructed in the needle elevation stand 60 in a way to be separable.

Some (e.g., a tip part) of the micro needles 50 that has risen at a predetermined height from an initial procedure location of the micro needle 50 by the elevation unit 40 penetrate the skin. In this state, as a radio frequency current is applied to the multiple micro needles 50 simultaneously, radio frequency stimuli may be simultaneously applied to a wide area for a short time. As a result, collagen can be synthesized and skin damage can be recovered because the activity of cells that constitute a derma tissue of the skin is increased.

A construction of the invasive high-frequency treatment device according to an embodiment of the present disclosure is described more specifically.

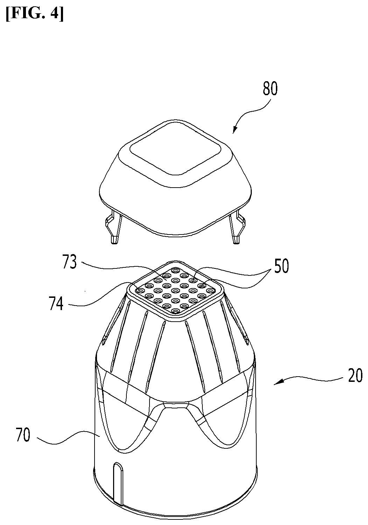

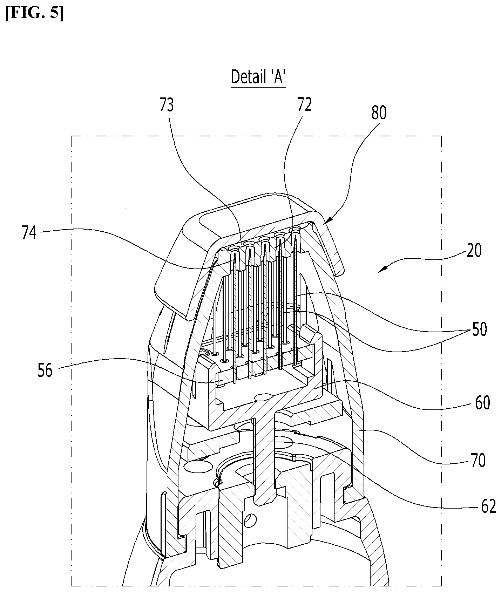

FIG. 4 is an enlarged perspective view illustrating the procedure tip illustrated in FIGS. 1 to 3. FIG. 5 is an enlarged cutaway perspective view of a portion "A" of the procedure tip illustrated in FIG. 3.

Referring to FIGS. 4 and 5, the procedure tip 20 includes the needle cap 70, the micro needles 50, and the needle elevation stand 60. The needle cap 70 may be an insulator, such as plastic, and may be formed in the form of a barrel having a width narrowed upward as illustrated in FIGS. 4 and 5. The needle cap 70 forms an appearance of the procedure tip 20 and accommodates the micro needles 50 in the accommodation space formed therein. Accordingly, the micro needles 50 can be protected against an external environment.

The multiple micro needles 50 may be constructed. The multiple micro needles 50 may be divided into a first group including first micro needles and a second group including second micro needles. Micro needles that belong to the same group may have the same polarity, and micro needles belonging to different groups may have opposite polarities. For example, if the first micro needle of the first group is a positive electrode (electrode (+)), the second micro needle of the second group may be a negative electrode (electrode (-)).

The first micro needle and the second micro needle may be alternately disposed. In this case, two micro needles 50 that are adjacent to each other may form a pair of opposite electrodes (the electrode (+) and the electrode (-)). Upon procedure, a voltage (+) may be applied to a group (i.e., the first group or second group) of the micro needles 50 that constitute the positive electrodes, and a voltage (-) may be applied to a group (i.e., the first group or second group) of the micro needles 50 that constitute the negative electrodes.

An end plate 72 having a flat plate shape may be formed at the top of the needle cap 70. A top surface (i.e., a surface exposed to the outside) of the end plate 72 becomes the procedure surface 73 that comes in direct contact with the skin upon procedure. Needle through holes 74 through which the multiple micro needles 50 are input and output simultaneously may be vertically formed in the end plate 72. In this case, the needle through holes 74 may each be formed to have a greater diameter than the micro needle 50. The multiple needle through holes 74 may be formed to correspond to the multiple micro needles 50, respectively.

For example, if the multiple micro needles 50 are arranged in a row, the needle through holes 74 formed in the end plate 72 of the needle cap 70 may also be formed in a row in accordance with the multiple micro needles 50. If the multiple micro needles 50 are arranged in the form of plural rows equal to or greater than two rows, the needle through holes 74 formed in the end plate 72 of the needle cap 70 may also be formed in plural rows equal to or greater than two rows so that the multiple micro needles 50 can be matched with the micro needles 50, respectively.

The needle elevation stand 60 may rise and fall within a set range by the elevation unit 40. As all of the multiple micro needles 50 are fixed to the needle elevation stand 60, the elevation of the multiple micro needles 50 may be synchronized with that of the needle elevation stand 60. More specifically, the multiple micro needles 50 may be stably mounted and fixed on the needle elevation stand 60 through a single needle substrate 56 in the state in which the multiple micro needles 50 have been mounted on the single needle substrate 56.

Although not illustrated, the procedure tip 20 may further include a guide block. In this case, the guide block may function to stably provide guidance to the elevation of the multiple micro needles 50 without the deformation of the multiple micro needles 50. The guide block may be fixed at a designated location within the needle cap 70. For example, the guide block may be fixed within the needle cap 70 between the needle elevation stand 60 and the end plate 72 of the needle cap 70 or may be connected to the needle elevation stand 60 within the needle cap 70 and moved along with the needle elevation stand 60.

In order to prevent an electrical short between the micro needles 50, the guide block may be made of a non-metal insulating material, such as rubber, silicon, or plastic. The guide block includes guide holes so that the guide holes are matched with the micro needles 50, respectively, and can provide guidance to the elevation of the micro needles 50, respectively. Accordingly, the micro needle 50 can stably penetrate the skin without being deformed even in resistance that is increased right before the micro needle penetrates the skin.

The procedure tip 20 is assembled with the handpiece 10 in a way to be separable as described above, and may be provided in several types in which the size of the procedure surface 73 or the arrangement of the micro needles 50 is different. For this reason, an operator (or user) may select a proper procedure tip 20 depending on a procedure portion and the state of the procedure portion and assemble the proper procedure tip 20 with the handpiece 10.

Upon procedure using the invasive high-frequency treatment device 1, the elevation unit 40 may rise and fall centering around a procedure zero point of the elevation unit 40. Specifically, upon procedure, the elevation unit 40 may advance from the procedure zero point to a predetermined location. Accordingly, the micro needles can enter the skin of an operator (or user). After the procedure, the elevation unit 40 may return to the procedure zero point. Accordingly, the micro needle may return its initial procedure location.

That is, the procedure zero point of the elevation unit 40 may correspond to the initial procedure location of the micro needle. The procedure zero point of the elevation unit 40 may be preset. For example, a preset procedure zero point of the elevation unit 40 may be a value by which the micro needle is moved to its proper initial procedure location.

For example, when an operator (or user) assembles the procedure tip 20 with the handpiece 10, the elevation unit 40 may be moved to a preset procedure zero point of the elevation unit 40, and the micro needles 50 may be moved to their initial procedure locations, respectively.

However, the initial procedure location of the micro needle, which corresponds to the preset procedure zero point of the elevation unit 40, may be changed due to several factors, such as the type (shape) of the procedure tip 20, the state of an assembly with the handpiece 10, an assembly state within the handpiece 10, or tolerance of the micro needle.

For example, although the elevation unit 40 is accurately placed at a preset procedure zero point of the elevation unit 40, an initial procedure location of the micro needle may be changed if the procedure tip 20 assembled with the handpiece 10 is changed. In this case, there is a problem in that a procedure effect is halved or a side effect may occur because the initial procedure location of the micro needle 50 is not uniformly disposed.

The invasive high-frequency treatment device 1 according to an embodiment of the present disclosure can consistently set the initial procedure location of the micro needle 50 regardless of the type of procedure tip 20, the state of an assembly with the handpiece 10, or an assembly state within the handpiece 10. In particular, the invasive high-frequency treatment device 1 according to an embodiment of the present disclosure may consistently set the initial procedure location of the micro needle 50 with respect to the procedure tip 20 (i.e., on the basis of the procedure tip 20).

FIG. 6 is a block diagram of components that are necessary to perform an operation of readjusting (or resetting) an initial procedure location and connection relations between corresponding components according to an embodiment of the present disclosure. Major components of the present disclosure are described with reference to FIG. 6.

Referring to FIG. 6, a predetermined location of the micro needle 50 may be determined with respect to the procedure tip 20. A procedure zero point of the elevation unit 40 may be determined to correspond to the determined location the micro needle 50. In particular, according to embodiments of the present disclosure, when the micro needle 50 is placed at a predetermined location on the basis of the procedure tip 20, a predetermined location of the micro needle 50 may be determined based on a change in the voltage generated in the micro needle 50 by causing a change in the voltage.

In an embodiment, the control circuit 30 may readjust the initial procedure location of the micro needle through a series of processes, such as control a rise of the micro needle -> detect a change in the voltage of the micro needle during the rise → stop the rise of the elevation unit when the detected change in the voltage is greater than a predetermined reference value → determine a procedure zero point of the elevation unit from the location of the micro needle when the rise of the elevation unit is stopped_{┘.}

In an initialization step of the invasive high-frequency treatment device 1, the control circuit 30 may determine a procedure zero point of the elevation unit 40. Specifically, the control circuit 30 may raise the micro needle 50 by controlling the elevation unit 40 in the initialization step, may detect a change in the voltage of the micro needle 50 during the rise of the micro needle 50, may stop the rise of the elevation unit 40 when the change in the voltage is greater than a predetermined reference value, and may determine a point at which the rise of the elevation unit 40 is stopped as a procedure zero point of the elevation unit 40.

Thereafter, in a procedure step of the invasive high-frequency treatment device 1, the control circuit 30 may control the elevation unit 40 to rise at a predetermined height from the procedure zero point of the elevation unit 40 so that the micro needle 50 can return to its predetermined initial procedure location.

In particular, according to embodiments of the present disclosure, when the micro needle 50 is placed at a predetermined location on the basis of the procedure tip 20, a change in the voltage of the micro needle 50 occurs. Accordingly, the procedure zero point of the elevation unit 40 may be determined on the basis of the occurrence of such a change in the voltage. Accordingly, the procedure zero point of the elevation unit 40 can be consistently maintained regardless of the type of procedure tip 20, the state of an assembly with the handpiece 10, or an assembly state within the handpiece 10.

If a conductor is placed at a predetermined location of the procedure tip 20, when the micro needle 50 comes into contact with the conductor, a change in the voltage of the micro needle 50 may occur. Accordingly, the micro needle 50 can be consistently determined on the basis of the procedure tip 20.

In FIG. 6, reference numeral 90 is a conductive plate disposed on the procedure surface 73 of the needle cap 70. In an embodiment, a method using the conductive plate 90 as in the example of FIG. 6 in detecting a change in the voltage of the micro needle 50 and determining an initial procedure location of the micro needle from a location of the elevation unit at which a change in the voltage is detected may be considered.

In the embodiment of the method using the conductive plate in FIG. 6, when power is turned on, the micro needle 50 rises by the elevation unit 40 according to a set control sequence and comes into contact with the conductive plate 90 on the procedure surface 73. At this time, an electrical short occurs. The control circuit 30 may detect the electrical short, and may determine the location of the elevation unit 40 corresponding to the location (i.e., the elevation location) of the micro needle 50 at the time point at which the electrical short is detected as a procedure zero point of the elevation unit 40. At this time, the location of the conductive plate 90 is relatively constant with respect to the procedure tip 20. Accordingly, the location (i.e., the procedure zero point of the elevation unit 40) of the micro needle 50 at the time point at which the electrical short is detected also become relatively consistent with respect to the procedure tip 20.

When the radio frequency treatment device is powered according to a control sequence set in the control circuit 30, the elevation unit 40 rises and simultaneously a predetermined voltage that determines a procedure zero point of the elevation unit 40 is applied to the micro needle 50. In this case, predetermined voltages that determine the procedure zero point may be applied to a first micro needle 52 and a second micro needle 54 having different polarities. When the first and second micro needles 52 and 54 come into contact with the conductive plate 90 simultaneously, a voltage of the micro needle is greatly changed because an electrical short in which a voltage (+) and a voltage (-) are directly connected.

In an embodiment, the control circuit 30 may detect a great change in the voltage attributable to the electrical short, and may determine the location of the elevation unit 40 corresponding to the location (i.e., the elevation location) of the micro needle 50 at a corresponding time point as a procedure zero point of the elevation unit 40. More specifically, when the detected change in the voltage is greater than a predetermined reference value, the control circuit 30 may immediately stop the rise of the elevation unit 40, may measure the location of the elevation unit 40 corresponding to the location of the micro needle 50 at a time point at which the rise of the elevation unit 40 is stopped, and may determine the measured location of the elevation unit 40 as the procedure zero point of the elevation unit 40.

A method of measuring the location of the elevation unit 40 may include several known methods of detecting a location in addition to a method using a control value (control duty) applied to the elevation unit 40 and a method of detecting a movement (rise) displacement of the elevation unit 40 in real time.

The control circuit 30 includes the control unit 32, the radio frequency generation unit 34, the voltage detection unit 36, and a relay 38. The control unit 32 controls the driving of the elevation unit 40. The radio frequency generation unit 34 generates a radio frequency from the input power and applies the radio frequency to the micro needle 50. Furthermore, the voltage detection unit 36 is disposed in parallel to the radio frequency generation unit 34 in a circuit manner, and plays a role of applying a voltage that determines the initial procedure location of the elevation unit 40 to the micro needle 50 and detecting a change in the voltage attributable to an electrical circuit.

When a change in the voltage attributable to the electrical short, which is detected by the voltage detection unit 36, is greater than a predetermined reference value, the voltage detection unit 36 outputs a detection signal indicating that the change in the voltage is greater than the predetermined reference value.

In response to the detection signal, the control unit 32 outputs an elevation control signal that stops the rise of the elevation unit 40 to the elevation unit 40.

In response to the elevation control signal, the elevation unit 40 stops the rise of the elevation unit 40, and outputs elevation location information indicating the location of the elevation unit 40 at that time.

The control unit 32 determines the location of the elevation unit 40 the rise of which is stopped, that is, the location of the elevation unit 40 when a change in the voltage of the micro needle 50 is greater than a reference value, as a procedure zero point of the elevation unit 40 based on the elevation location information.

Furthermore, the control unit 32 may correct an initial procedure location of the micro needle based on a preset location correction value (or offset value). The preset location correction value is a value that is previously determined depending on a shape of the procedure tip 20, and functions to additionally correct the procedure zero point of the elevation unit 40 based on the detected change in the voltage.

In this case, the location correction value (or offset value) may be set as a negative (-) value so that the initial procedure location of the micro needle 50 is determined at a location that is downward moved by a predetermined distance from a location at which the micro needle 50 comes into contact with the conductive plate 90 (i.e., move the micro needle in a direction in which the micro needle enters the needle cap). The location correction value may be set in the range of -0.5 mm to -0.1 mm, but is not specially limited to the range because the range of the location correction value may be different depending on the specifications of the procedure tip 20.

For example, when a set location correction value (or offset value) is -0.2 mm, the control unit 32 may determine a location that is moved 0.2 mm downward (i.e., a direction in which the micro needle enters the needle cap) on the basis of a location at which an electrical short occurs because the risen micro needle 50 comes into contact with the conductive plate 90 as the procedure zero point of the elevation unit 40.

The relay 38 may be disposed between the micro needle 50, and the radio frequency generation unit 34 and the voltage detection unit 36. In an embodiment, the relay 38 may electrically connect the micro needle 50 and the radio frequency generation unit 34 under the control of the control unit 32. Furthermore, the relay 38 may electrically connect the micro needle 50 and the voltage detection unit 36. That is, the relay 38 may be driven to alternatively connect one of the radio frequency generation unit 34 and the voltage detection unit 36 to the micro needle 50 under the control of the control unit 32.

In an embodiment, the relay 38 may switch to a first location in the initialization step. Furthermore, the determination of the initial procedure location of the micro needle 50 is completed through a series of such pieces of processing. When the procedure step is entered, the relay 38 may switch to a second location. The first location may be a location at which the micro needle 50 and the voltage detection unit 36 are electrically connected. The second location may be a location at which the micro needle 50 and the radio frequency generation unit 34 are electrically connected.

In the method using the conductive plate, as in the example of FIG. 6, the conductive plate 90 may be installed within the tip cover 80 that is detachably coupled to the needle cap 70. In this case, the conductive plate 90 may be installed within the tip cover 80 at a height at which a surface (i.e., the bottom of the conductive plate 90 in FIG. 6) of the conductive plate 90, which faces the procedure surface 73, may closely come into surface contact with the flat procedure surface 73 when the tip cover 80 covers the procedure surface 73 of the needle cap 70 (i.e., when the tip cover is coupled to a designated location of the needle cap).

Although not illustrated, the conductive plate 90 has a form of an adhesive tape, and may be constructed in the form of an expendable part that is attached and fixed to the procedure surface 73. In addition, a location, form, or coupling structure of the conductive plate 90 may be variously changed depending on the size or shape of the procedure tip 20 or an arrangement structure of the micro needles 50. Accordingly, a construction of the conductive plate 90 capable of implementing an electrical short of the micro needle 50 that protrudes from the procedure surface 73 may be applied without special limitation.

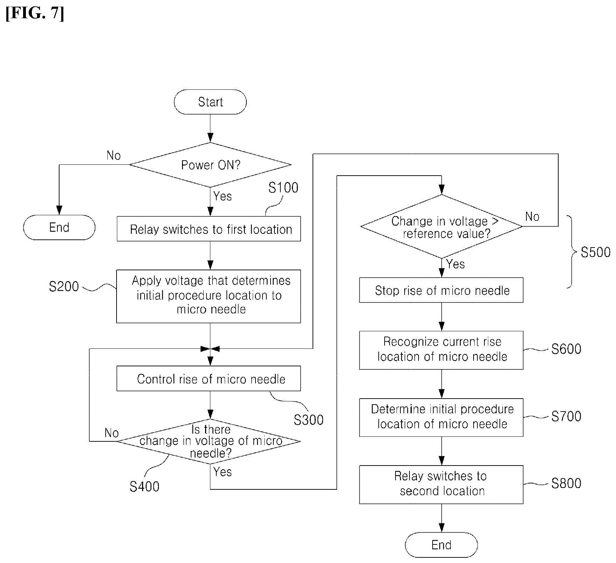

Hereinafter, a process of determining the procedure zero point of the elevation unit 40, which is performed in the invasive high-frequency treatment device according to an embodiment of the present disclosure, is simply described with reference to the flowchart of FIG. 7. The components illustrated in FIG. 6 are described with reference to corresponding reference numerals for convenience of description.

FIG. 7 is a flowchart for describing a process of readjusting (or resetting) an initial procedure location of the micro needle, which is performed in the invasive high-frequency treatment device according to an embodiment of the present disclosure.

Referring to FIG. 7, in the initialization step, the control unit 32 of the control circuit 30 outputs a switch command to the relay 38 (S100). For example, the initialization step may be a step that is entered after the turn-on of the invasive high-frequency treatment device 1, the coupling of the procedure tip 20 with the handpiece 10, a manipulation of a separate dedicated button, and then a preset procedure cycle/number, but the present disclosure is not limited thereto. A first location may be a location at which the micro needle 50 and the voltage detection unit 36 are electrically connected.

When the relay 38 switches to the first location, the control unit 32 applies a voltage that determines an initial procedure location of the micro needle 50 to the micro needle 50 through the voltage detection unit 36 (S200). Furthermore, the control unit 32 outputs driving commands that raise the micro needles 50 simultaneously or with some time difference to the elevation unit 40 (S300). Accordingly, the micro needle 50 rises. While the micro needle 50 rises, the voltage detection unit 36 detects (or monitors) a change in the voltage of the micro needle 50 in real time (S400).

When there is no change in the voltage of the micro needle 50, the control unit 32 continuously raises the micro needle 50 by controlling the elevation unit 40 (refer to a direction "No" in step S400). In contrast, when a change in the voltage of the micro needle 50 during the rise is detected and the change is greater than a predetermined reference value, the voltage detection unit 36 immediately transmits corresponding information to the control unit 32. The control unit 32 stops the rise of the micro needle 50 by outputting a rise stop command to the elevation unit 40 at a time point when the control unit 32 receives the corresponding information (S500).

For example, if the method using the conductive plate 90 is used as illustrated in FIG. 6, the voltage detection unit 36 may apply a predetermined voltage (+) and a predetermined voltage (-) to the first micro needle 52 and the second micro needle 54 having different polarities, may detect a great change in the voltage attributable to an electrical short that occurs because the first micro needle 52 and the second micro needle 54 that have risen come into contact with the conductive plate 90 simultaneously, and may transmit corresponding information to the control unit 32.

If the detected change in the voltage of the micro needle 50 is greater than the predetermined reference value, when the rise of the micro needle 50 is stopped in response to the rise stop command that is output from the control unit 32 to the elevation unit 40, the control unit 32 recognizes the location of the elevation unit 40 at a corresponding time point, that is, a time point at which the rise of the micro needle 50 is stopped (S600). Furthermore, the control unit 32 determines the recognized location value as a procedure zero point of the elevation unit 40 (S700).

When the determination of the procedure zero point of the elevation unit 40 is completed through a series of such pieces of processing, the control unit 32 controls the invasive high-frequency treatment device 1 to operate in the procedure step. In the procedure step, the control unit 32 outputs a command that changes the location of the relay 38 to a second location (i.e., a location at which the micro needle is connected to the radio frequency generation unit) to the relay 38 (S800). Accordingly, as the relay 38 switches to the second location, the invasive high-frequency treatment device 1 may enter the procedure step.

According to the embodiments of the present disclosure, it is possible to consistently set an initial procedure location of the micro needle regardless of a change in the initial location of the micro needle attributable to the type of procedure tip, the state of an assembly with the handpiece, or an assembly state within the handpiece. Accordingly, several conventional problems (e.g., inaccurate control over a procedure depth and a reduced procedure effect or the occurrence of a procedure side effect according to the inaccurate control, displeasure or the cause of a pain because the micro needle protrudes over the procedure surface in a procedure standby state), which occur because the micro needle is disposed to fall outside a planned initial procedure location, can be solved.

The above description is merely a description of the technical spirit of the present disclosure, and those skilled in the art may change and modify the present disclosure in various ways without departing from the essential characteristic of the present disclosure. Accordingly, the embodiments described in the present disclosure should not be construed as limiting the technical spirit of the present disclosure, but should be construed as describing the technical spirit of the present disclosure. The range of the technical spirit of the present disclosure is not restricted by the embodiments. The range of protection of the present disclosure should be construed based on the following claims, and all of technical spirits within an equivalent range of the present disclosure should be construed as being included in the scope of rights of the present disclosure.

**[Description of reference numerals]**

| | | | |
|---|---|---|---|
| 1: | invasive high-frequency treatment device | | |
| 10: | handpiece | 20: | procedure tip |
| 30: | control circuit | 32: | control unit |
| 34: | radio frequency generation unit | | |
| 36: | voltage detection unit | 38: | relay |
| 40: | elevation unit | 42: | actuator |
| 44: | elevation shaft | 50: | micro needles |
| 52: | first micro needle | 54: | second micro needle |
| 56: | needle substrate | 60: | needle elevation stand |
| 62: | shaft unit | 70: | needle cap |
| 72: | end plate | 73: | procedure surface |
| 74: | needle through holes | 80: | tip cover |
| 90: | conductive plate | | |

## Claims

1. An invasive high-frequency treatment device comprising:
multiple micro needles comprising a first micro needle and a second micro needle;
an elevation unit configured to raise the micro needle; and
a control circuit configured to control the elevation unit,
wherein the control circuit
determines a procedure zero point of the elevation unit corresponding to the multiple micro needles in an initialization step of the invasive high-frequency treatment device, and
controls the elevation unit to rise at a predetermined height from the procedure zero point in a procedure step of the invasive high-frequency treatment device.

2. The invasive high-frequency treatment device of claim 1, wherein in the initialization step, the control circuit
raises the multiple micro needles by controlling the elevation unit,
detects a change in a voltage of at least one of the multiple micro needles, and
determines, as the procedure zero point, a location of the elevation unit corresponding to a location of the micro needle when the change in the voltage is greater than a predetermined reference value based on the results of the detection.

3. The invasive high-frequency treatment device of claim 2, further comprising a conductive plate disposed on the multiple micro needles.

4. The invasive high-frequency treatment device of claim 3, further comprising a needle cap disposed over the multiple micro needles, wherein the conductive plate is constructed in a form of an adhesive tape that is attached and fixed to a procedure surface of the needle cap.

5. The invasive high-frequency treatment device of claim 3, further comprising a needle cap disposed on the multiple micro needles, wherein the conductive plate is installed within a tip cover detachably coupled to the needle cap.

6. The invasive high-frequency treatment device of claim 1, wherein the control circuit comprises:
a control unit configured to control the elevation unit;
a radio frequency generation unit configured to generate radio frequency power from input power and to apply the generated radio frequency power to the micro needles; and
a voltage detection unit configured to apply a voltage that determines an initial procedure location of to the micro needle to the micro needle and to detect a change in the voltage.

7. The invasive high-frequency treatment device of claim 6, wherein:
the voltage detection unit outputs a detection signal indicating that the change in the voltage is greater than the predetermined reference value,
the control unit outputs, to the elevation unit, an elevation control signal indicative of a stop of the rise of the elevation unit in response to the detection signal,
the elevation unit stops the rise of the elevation unit in response to the elevation control signal and outputs elevation location information indicative of a current location of the elevation unit, and
the control unit determines the current location of the elevation unit the rise of which is stopped as the procedure zero point based on the elevation location information.

8. The invasive high-frequency treatment device of claim 1, wherein the control circuit reads a location correction value stored therein and corrects the procedure zero point based on the read location correction value.

9. The invasive high-frequency treatment device of claim 6, wherein the control circuit further comprises a relay configured to alternatively connect one of the radio frequency generation unit and the voltage detection unit to the micro needle.

10. The invasive high-frequency treatment device of claim 9, wherein the relay
switches to a first location at which the micro needles and the voltage detection unit are electrically connected in the initialization step, and
switches to a second location at which the micro needles and the radio frequency generation unit are electrically connected in the procedure step.

11. The invasive high-frequency treatment device of claim 1, wherein the control circuit controls the invasive high-frequency treatment device to perform the initialization step when the multiple micro needles and the control circuit are newly electrically connected.
